# EUROPEAN PATENT APPLICATION

(11) **EP 1 584 924 A2**
(43) Date of publication of application: **12.10.2005**
(21) Application number: 05252132.5
(22) Date of filing: 05.04.2005
(51) Int. Cl.: G01N 33/497

(54) **Breathalyser**

(30) Priority: 07.04.2004 DE 102004017068
(71) Applicant: Dräger Safety AG & Co KGaA, 23560 Lübeck (DE)
(72) Inventor: Stock, Burkhard, 23562 Lübeck (DE); Kater, Theodor, 23564 Lübeck (DE)
(74) Representative: Greenwood, John David

(57) **Abstract**

A breathalyser with a pressure sensor (2) for recording a signal representative of the breathing air flow, an electrochemical sensor (1) for recording a signal representative of the alcohol content of the breathing air, and with evaluation devices for processing the signals and determining the breath alcohol concentration. A simple construction is provided with high measuring accuracy by virtue of the pressure sensor (1) and the electrochemical sensor (2) being connected to a delta-sigma analogue-digital converter (8), which converts the sensor signals directly to digital measured values for determining the alcohol concentration. This renders the pre-amplifiers previously required superfluous, which presents a number of advantages.

## Description

This invention relates to a breathalyser with a pressure sensor for recording a signal that is representative of the breathing air flow, with an electrochemical sensor for recording a signal representative of the alcohol content of a breathing air sample and with evaluation devices for processing the signals and determining the breath alcohol concentration.

Such breathalysers are known, for example, from DE 43 273 312 C2 and US patent 4,770,026. The pressure sensor is required for generating a signal that is representative of the respiratory volumetric flow. This is required on the one hand to be able to monitor the uninterrupted exhalation of the subject. On the other hand, the respiratory volumetric flow signal is integrated so that a predetermined minimum respiratory volume required for a reliable measured result can be determined, since a sufficient proportion of breathing air from the depths of the lungs is required to be able to determine the blood alcohol concentration from the breath alcohol concentration.

The electrochemical sensor is subjected to a predetermined respiratory volume sample. On the electrodes of the electrochemical sensor ethanol is selectively converted, and an electrical current is generated which slowly fades after a rapid increase, corresponding to the tapering reaction. The sensor current is integrated for the predetermined period of time and the total load is extrapolated from this integrated load value, from which total load the breath alcohol concentration may be derived.

In the known breathalysers the signals from the pressure sensor and the electrochemical sensor are all amplified initially in a pre-amplifier or operating amplifier, then fed to an analogue-digital converter with a range of 10 bits or 12 bits. The analogue processing of the sensor signals before their digitalisation presents various disadvantages.

The electrochemical sensor is connected to a current-voltage converter which is formed by an operating amplifier fed back via a resistance to one of its inputs. When the supply voltage is turned on, i.e. when the device is switched on, the operating amplifier passes through states of non-equilibrium. This causes a current to be transmitted for a short time to the electrochemical sensor, resulting in an accumulation of potential in the sensor. Since it can take approx. 20 seconds for this potential to reduce again, this results in a corresponding waiting time until the device is ready for use after being switched on.

If the device is not switched on the sensor is not short-circuited either. Because of thermal influences or gases in the ambient air, a potential can build up which first needs to reduce again after the device is switched on, which again leads to a longer waiting time.

A further disadvantage of the known measuring devices lies in the fact that several analogue-digital channels with different amplifications are often required to be able to cover the required dynamic range, because for integration of the signals of the electrochemical sensor the evaluation devices require a resolution of 7 bits. The ratio of the largest signal (5 per thousand at 50°C) to the smallest (0.1 per thousand at -5°C) is approx. 1000/1 (10 bits). In the 10 bit analogue-digital converters typically used the signal from the electrochemical sensor must therefore be divided into several analogue-digital channels with different amplifications in order to be able to cover the dynamic range. This is associated with a number of disadvantages, namely a greater component requirement, increased energy consumption and complicated signal processing.

A further disadvantage of the system of prior art for processing the analogue signals in pre-amplifiers resides in the fact that electromagnetic radiation may influence the measurements. Electromagnetic radiation gives rise to faults in the input circuits of the amplifiers, which are then also amplified and give rise to incorrect measured results.

The following must be pointed out with regard to the signal processing of the pressure sensor. The pressure sensor measures the dynamic pressure generated in the nozzle. This dynamic pressure is then converted to a respiratory volumetric flow. Since the smallest recordable respiratory volumetric flow should be approx. 3 I/min, but the highest approx. 50 I/min., this results in a very wide dynamic range. Furthermore, pressure sensors are constructed in the form of a bridge circuit which supplies a static offset voltage even without pressure. This static offset voltage limits the possible amplification of the pressure signal to a factor of approx. 100 in real systems, which in turn results in an insufficient digital resolution at small respiratory volumetric flows. Since most calibrations supply only a low current, special nozzles are then required for calibration. As in the case of the electrochemical sensor, the amplifiers used for the pressure sensor are also susceptible to electromagnetic faults.

The present invention is as claimed in the claims.

Embodiments of the present invention can be of simple construction and provide high measuring accuracy.

According to the present invention, analogue pre-processing of the signals from the electrochemical sensor and pressure sensor can be dispensed with. The sensor signals are fed directly, without pre-amplification, to a high resolution delta-sigma analogue-digital converter, thus pre-amplifiers may be dispensed with altogether. This also eliminates the above-mentioned problems arising from analogue pre-amplification.

In an advantageous embodiment an individual delta-sigma analogue-digital converter with two inputs is used, the delta-sigma analogue-digital converter being controlled so that alternately it first processes the signals of one input, then those of the other input. In an advantageous embodiment a 24 bit delta-sigma analogue-digital converter is used whose output signals are used by a microprocessor in the evaluation devices to determine a value for the breath alcohol concentration, and hence a measure of the blood alcohol concentration, from the measured signal from the electrochemical sensor.

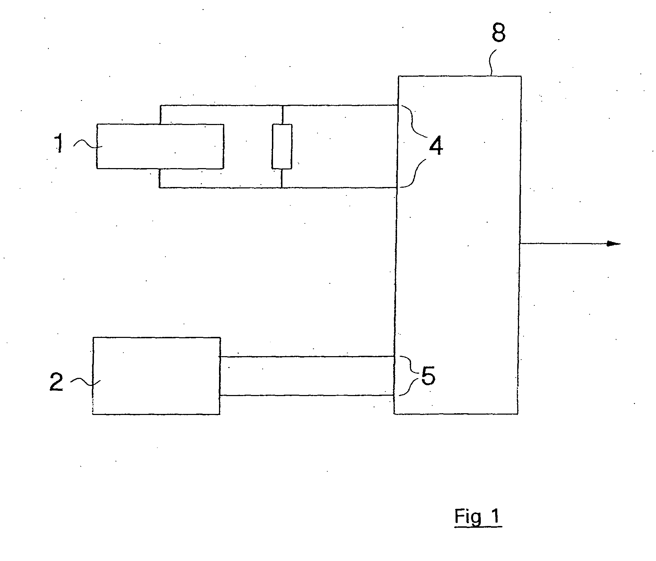

The present invention will now be described, by way of example only, with reference to an embodiment of the single figure, Figure 1, which is a block schematic diagram of a part of the breathalyser according to the invention.

The breathalyser of Figure 1 includes an electrochemical sensor 1 and a pressure sensor 2, which are arranged by a known method in an alcohol test meter, e.g. the Alcotest meter 7410 of the applicant of this patent application described in Dräger Volume 346 (January-May 1990).

According to the present invention, the differential signals from electrochemical sensor 1 and pressure sensor 2 are fed directly, i.e. without pre-amplification, as analogue signals to differential inputs 4 and 5 of delta-sigma analogue-digital converter 8. Such a high resolution delta-sigma analogue-digital converter 8, e.g. such a 24 bit converter, can evaluate the sensor signals directly in the nV range and transmit them as digitalised data values to a microprocessor, (not shown), which then carries out the further calculations and evaluations.

As shown, the differential cables from electrochemical sensor 1 are connected by a low ohm resistance so that no interfering potentials are able to build up in electrochemical sensor 1. The problems associated with the state of the art, such as lack of dynamics and resolution and too high a sensitivity to electromagnetic interferences, can be wholly avoided with the construction described. Moreover, the breathalyser can be designed to be more compact because fewer components are present.

## Claims

1. A breathalyser, with a pressure sensor for recording a signal representative of the breathing air flow, with an electrochemical sensor for recording a signal representative of the alcohol content of the breathing air, and with evaluation devices for processing the signals and determining the breath alcohol concentration, **characterised in that** the pressure sensor (2) and electrochemical sensor (1) are connected to a delta-sigma analogue-to-digital converter means (8) which is arranged to convert the sensor signals directly to digital measured values for determining the alcohol concentration.

2. The breathalyser according to claim 1, including a single delta-sigma analogue-to-digital converter (8) with at least two inputs (4, 5), and in which, the pressure sensor (2) is connected to the first input (5) and the electrochemical sensor (1) is connected to the second input (4), and the delta-sigma analogue-to-digital converter (8) is arranged to process, alternately, the signals of the first and second inputs.

3. The breathalyser according to claim 1 or 2, in which the analogue-to-digital converter (8) is a 24 bit delta-sigma analogue-to-digital converter.
